# EUROPEAN PATENT SPECIFICATION

(11) **EP 0 819 179 B1**
(45) Date of publication and mention of the grant of the patent: **13.09.2006**
(21) Application number: 96907599.3
(22) Date of filing: 20.03.1996
(51) Int. Cl.: C12Q 1/04, C12N 1/00, C12Q 1/06, C12M 1/00

(54) **MICROBIAL MONITORING**
MIKROBE-MONITORING
CONTROLE MICROBIEN

(30) Priority: 20.03.1995 GB 9505573; 25.09.1995 GB 9519516
(43) Date of publication of application: 21.01.1998
(73) Proprietor: Echa Microbiology Limited, Titan Road Cardiff CF24 5EJ (GB)
(72) Inventor: HILL, Edward, Charles, Cardiff CF11 6QH (GB)
(74) Representative: Cawdell, Karen Teresa
(86) International application number: PCT/GB1996/000673
(87) International publication number: WO 1996/029428

(56) References cited:
- US-A- 3 935 067

## Description

The present invention relates to microbial monitoring and, in particular, to a method and apparatus for microbial monitoring which may be used on site.

The quantitative and qualitative determination of the number of viable micro-organisms, such as bacteria, present in samples (such as fluids) liable to microbial spoilage or contamination has conventionally been carried out by stimulating any micro-organisms present in the sample to reproduce in or on a suitable growth medium, such as nutrient agar or the like, each individual viable microbial particle reproducing to form a respective visible colony in or on the growth medium.

The growth medium is typically inoculated with the sample to be tested, usually by a standard plating method, which involves spreading a liquid or solid sample on the surface of the growth medium or pressing the growth medium against a test surface believed to be contaminated with micro-organisms; a similar method comprises dipping a small paddle coated with the growth medium into a liquid sample and allowing the paddle to drain. On incubation, visible colonies form on the growth medium, which colonies can be monitored after a defined incubation period (such as one to five days) to provide a count or estimate of colony numbers. This count or estimate is indicative of the number of viable microbial particles in the original sample. The method just described requires substantially sterile conditions so as to inhibit contamination of the sample from micro-organisms not arising from the sample to be tested. The plating method described is generally limited to laboratory use. The paddle method is not strictly quantitative, is relatively slow, and is only suitable for monitoring of samples which have relatively high levels of microbial contamination, because only small samples can be applied to the surface of the growth medium.

Another known method comprises inoculation of a growth medium (such as nutritive agar) which has been liquefied (melted) by heating, and then agitated to disperse the sample in the medium, and then cooled to form a substantially rigid gel. Any micro-organisms present in the sample will therefore become dispersed within the growth medium, and can be incubated to grow into colonies which can be monitored in a similar manner to that described above for the plating method.

The method just described is suitable for monitoring of larger samples (typically 1 ml of sample may be introduced into 15 ml of molten agar in a Petri dish) and of samples having a relatively low level of microbial contamination. However, the method suffers from the disadvantage that it is necessary to expose any micro-organisms in the sample to the shock of contact with molten growth medium, which is typically at approximately 50°C; this can substantially inhibit reproduction and subsequent development of microbial colonies in the growth medium. In addition, this method is not suitable for on site testing (for example on a food production line, in a fuel tank or in a ship's cargo hold or the like) because it requires a molten growth medium, and its use is therefore generally confined to the laboratory.

Therefore, it is the purpose of the present invention to alleviate such difficulties and to provide a method and apparatus for monitoring micro-organisms, which method and apparatus can be used for both on site testing of a sample and for laboratory use, and which method and apparatus are quick and easy to use.

According to a first aspect of the invention there is provided a method of monitoring micro-organisms in a sample, which method comprises introducing the sample into a sealable container together with a nutritive gel medium suitable for growth of micro-organisms, agitating the container so as to liquefy the gel and substantially disperse microorganisms present in the sample in the gel medium, allowing the gel medium to set in the container, incubating the medium and monitoring the growth and/or activity of any micro-organisms within the gel medium, wherein the gel medium is thixotropic or pseudoplastic; such that agitation causes liquefaction thereof Incubation is at a suitable temperature for the micro-organisms.

It is an advantageous feature of the present invention that the micro-organisms may be easily dispersed within the gel medium by simple agitation, and are then fixed in spatial distribution when the gel medium has been allowed to set, because of the thixotropic or pseudoplastic nature of the medium.

The method according to the invention does not require molten agar or the like, and is particularly suitable for on site testing, because of the ease with which the sample can be dispersed within the gel medium and subsequently monitored.

According to a second aspect of the present invention, there is provided apparatus for monitoring growth of micro-organisms, which apparatus comprises a sealable container having a nutritive gel medium suitable for growth of micro-organisms therein, the gel medium comprising a thixotropic or pseudoplastic gel or a material capable of forming a thixotropic or pseudoplastic gel.

The gel medium may be an aqueous medium containing one or more thixotropic or pseudoplastic agents, or both. Examples of suitable thixotropic or pseudoplastic agents include the following:
natural gums derived, for example, from exudates or extracts of plants, animals or micro-organisms, such as tragacanth, guar, gum arabic, carrageenan, starch, dextrins, algin, pectin, starch, ghatti, locust bean, carob, tara, aloe.chia, flaxseed, quince seed, psyllum seed, tamarind, corn fibre, karaya, chitin, gelatin and xanthan. The thixotropic or pseudoplastic agents may also be semi-synthetic polymers such as carboxymethylcellulose, hydroxyethyl cellulose, hydroxypropyl cellulose, ethyl cellulose, hydroxymethyl cellulose, hydroxyalkyl cellulose or alginates. Synthetic gel material, such as polyethylene oxide polymers and silica gels may also be used as the thixotropic or pseudoplastic agent.

The thixotropic or pseudoplastic agent may be provided in substantially anhydrous form, and be reconstituted to form a gel by the addition of water prior to use in the method according to the invention.

The resulting gel medium is preferably transparent or translucent to visible light in order to permit optical monitoring thereof.

Preferably, the gel medium used in the method and apparatus according to the invention contains an indicator of microbial metabolism, such an indicator being preferably one which reacts with microbial cells or colonies in the medium to generate a colour which is readily distinguishable from the usual colour of the gel. The indicator may comprise, for example, a tetrazolium salt, a chemical indicator of microbial respiration which undergoes a colour change from colourless to blue/red/purple in the presence of viable micro-organisms, or a fluorescent compound. Further indicators may be used, additionally or alternatively, for example, in order to detect microbial products of respiration or metabolism, enzymes, sulfides, acids and/or alkalies. Additional chemicals may be included to enhance the performance of indicators, for example electron coupling agents such as menadione.

Indicators of microbial activity can advantageously provide a rapid indication of such activity, even within 10 to 20 minutes of incubation. The method according to the invention can therefore be used to provide either a qualitative or quantitative indication of the number of micro-organisms present in the sample.

Where a sample to be monitored according to the invention is heavily contaminated with micro-organisms, and/or where incubation has proceeded for a relatively long time, the colonies in the gel medium may overlap substantially, so that it may not be possible to count the resulting colonies. However, the method according to the invention permits a quantitative indication of the number of micro-organisms present in a heavily contaminated sample because the intensity of the colour change of the colonies in the gel medium may provide a measure of the degree of microbial contamination (for example, the perceived colour of the gel medium may increase in intensity when the quantity of micro-organisms is in excess of 1000 per ml). The intensity of the colour change in the gel medium on growth of the colonies can be compared with calibration standards to provide an indication of the numbers of micro-organisms present.

The gel medium may advantageously contain specific chemicals which substantially inhibit growth of certain groups of micro-organisms, or nutrients which selectively encourage the growth of others. Similarly various nutrients or chemicals may be incorporated into the gel to permit selective growth of micro-organisms having particular characteristics, for example those micro-organisms having the ability to metabolise hydrocarbons or those microbes which can reduce sulfur compounds.

The sealable container may be a transparent vial or the like, containing the gel medium. The container may, for example, be provided with a screw top. After liquifying the gel by agitation with the sample, solidification can take place in various configurations, for example as a deep gel or a thin film. The configuration of the set gel may be selected according to the preferred growth conditions of the micro-organisms being monitored.

The sample may be mixed directly with the gel medium in the container when, for example, the sample is an aqueous liquid. Alternatively, the sample may be pre-treated and then mixed with the gel medium in the container.

When the sample is derived from a non-aqueous based liquid, such as an oil or the like, the sample may be directly mixed with the gel medium. Alternatively the method according to the invention may further comprise a pre-treatment step, which may for example comprise addition of the liquid to an aqueous liquid, so that the micro-organisms are advantageously transferred to the aqueous liquid as the oil forms a separate phase distinct from the aqueous liquid. The aqueous liquid, which at that point includes the micro-organisms, can then be added to the gel medium in the container for incubation and monitoring of microbial growth. Similarly, where the sample comprises a piece of food or the like, the sample may be added directly to the gel medium or alternatively may be broken down or dissolved in an aqueous liquid.

The sample may furthermore be derived from use of a swab, such as, for example, a cotton wool or alginate swab.

The method according to the invention is versatile and can be used in a wide range of applications where an indication of microbial activity is required, particularly for on site investigations of microbial contamination. Such applications include the following:
a) for testing of microbial contamination of, for example, metal-working fluids, process water, fuels and other industrial fluids;
b) in the food and drink industry, on food production lines where it is necessary to test for food-borne pathogens and spoilage micro-organisms in process fluids, on equipment and finished products;
c) in the production of potable and bottled water;
d) in medical applications, for example, in tests for specific micro-organisms, such as, for example, Pseudomonas strains in hospitals (such micro-organisms being responsible for many hospital acquired infections).

The invention may be more clearly understood from the following examples of embodiments thereof given by way of example only and with reference to the accompanying drawings wherein:
Figure 1 illustrates a glass container approx. 100 x 15mm having a pseudoplastic gel medium therein and an indicator of microbial respiration, prior to the addition of a sample;
Figure 2 shows the container of Figure 1 with a 1ml sample containing bacteria dispersed therein, after shaking and allowing the gel to set with the container vertical;
Figure 3a illustrates a possible range of results obtained after a ten-minute incubation period;
Figure 3b illustrates a possible range of results after a 24 hour incubation period;
Figure 4 illustrates an 85 x 25mm glass container having a pseudoplastic gel medium therein and an indicator of microbial respiration, prior to addition of a sample;
Figure 5 illustrates the container of Figure 4 having a sample containing bacteria dispersed therein, after shaking and allowing the gel to set as a horizontal film;
Figure 6 illustrates a possible range of results after a 24-hour incubation period, with the containers being laid horizontally and viewed from above.

### Example 1

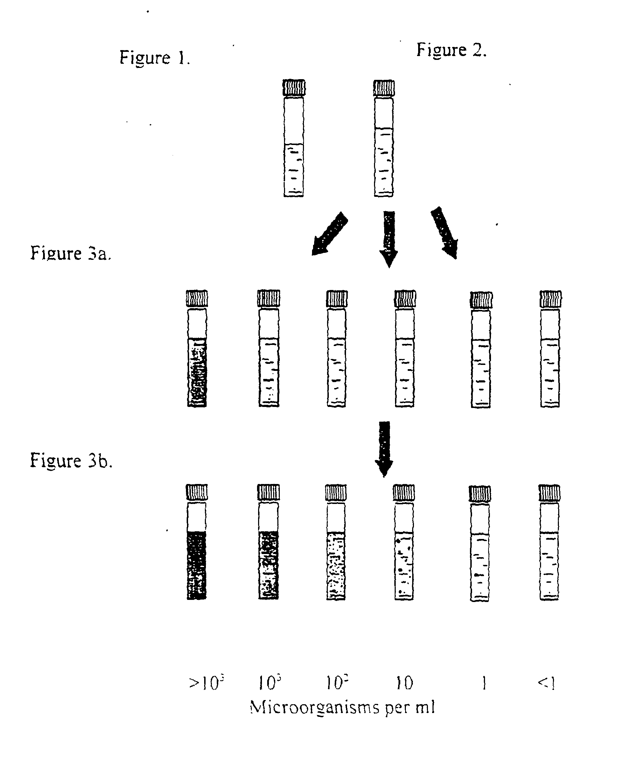

1ml of a sample containing bacteria was introduced into the gel medium in the glass containers of Figure 1. The nutrients incorporated into the gel medium are digests of casein and meat and sodium succinate. The gelling agent is xanthan and the indicator of microbial activity p.iodo.nitro.tetrazolium violet. Sodium chloride is incorporated to adjust the osmotic pressure.

After 10 minutes incubation the red/purple coloration which varies in intensity for those samples containing in excess of c. 1000 micro-organisms per ml as shown in Figure 3a, was compared to calibration standards to semi-quantitatively determine the numbers of viable microbes present, provided the number is large.

After 24 hours incubation a red/purple coloration which varies in intensity for those samples containing in excess of c. 1000 micro-organisms per ml as shown in Figure 3b, was compared to calibration standards to semi-quantitatively determine the numbers of viable microbes present.

A spotted appearance occurs for samples containing 1 to 1000 micro-organisms per ml. The number of spots (colonies) can be counted or estimated and the figure used to deduce the numbers of micro-organisms originally present in the sample.

Using a sample aliquot of 0.01ml, 0.1 ml or 1 ml or tenfold dilutions would alter the calibration by an order of magnitude, i.e. colour changes or numbers of microbial colonies counted or estimated would respectively refer to 0.01, 0.1 ml and 1 ml.

Large numbers of micro-organisms present in a sample will yield merging or overlapping colonies (Figure 3b); this can be overcome if so desired by diluting the sample with a sterile aqueous fluid before testing it or by using a smaller aliquot of sample.

### Example 2

The glass container is approx. 85 x 25mm and a sample containing bacteria is introduced in the same manner as in Example 1. The nutrients are digests of casein and meat, glucose and sodium pyruvate; the gelling agent is carrageenan and the indicator of microbial activity is p.iodo.nitro.tetrazolium violet; sodium chloride is incorporated to adjust the osmotic pressure. This formulation is suited for the detection of bacteria, moulds and yeasts; after adding a sample and agitating to mix and liquify the compounds, the gel is allowed to set as a horizontal film.

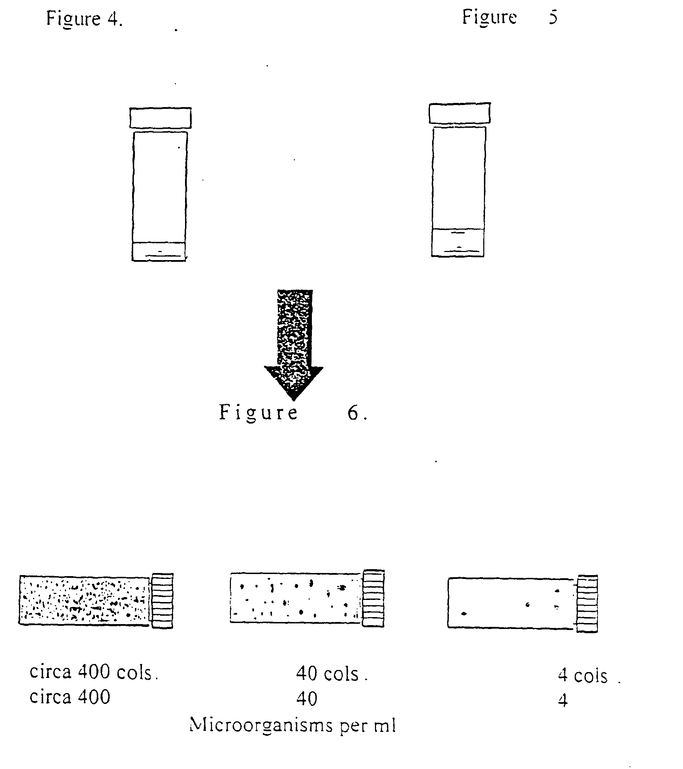

Typical results with this composition are illustrated in Figures 4,5 and 6.

After 24 hours incubation a red/purple coloration which varies in intensity can be seen which is similar to those results of Example 1.

## Claims

1. A method of monitoring micro-organisms in a sample, which method comprises introducing the sample into a sealable container together with a nutritive gel medium suitable for growth of micro-organisms, agitating the container so as to liquefy the gel and substantially disperse micro-organisms present in the sample in the gel medium, allowing the gel medium to set in the container, and monitoring the growth and/or activity of any micro-organisms within the gel medium, wherein the gel medium is thixotropic or pseudoplastic, such that agitation causes liquefaction thereof.

2. A method according to claim 1, wherein said gel medium comprises a semi-synthetic or synthetic polymer, or a mineral, as gelling agent.

3. A method according to claim 2, wherein said gelling agent comprises one or more of carboxymethylcellulose, hydroxyethyl cellulose, hydroxypropyl cellulose, ethyl cellulose, hydroxymethyl cellulose, hydroxyalkyl cellulose, an alginate, a polyethylene oxide polymer or a silica gel.

4. A method according to claim 1 or 3, wherein said gel medium comprises, as gelling agent, a gum derived from a natural source.

5. A method according to any preceding claim wherein said gel medium is aqueous.

6. A method according to claim 4,wherein said gum comprises one or more of guar, gum arabic, carrageenan, starch, dextrin, algin, tragacanth, pectin, ghatti, locust bean, carob, tara, aloe.chia, flax seed, quince seed, psyllum seed, tamarind, corn fibre, karaya, chitin, gelatin and xanthan.

7. A method according to any of claims 4 to 6, wherein said thixotropic or pseudoplastic agent is provided in substantially anhydrous form and is transformed to the gel medium by the addition of water.

8. A method according to any preceding claim, wherein said gel medium is transparent or translucent to visible light.

9. A method according to any preceding claim, wherein said gel medium comprises an indicator of microbial metabolism (such as a tetrazolium salt).

10. A method according to claim 9, wherein said indicator is capable of reacting with microbial cells or colonies in the medium to generate a colour which is readily distinguishable from the usual colour of the gel.

## Patentansprüche

1. Verfahren zur Überwachung (Monitoring) von Mikroorganismen in einer Probe, umfassend das Einführen der Probe in einen verschließbaren Behälter gemeinsam mit einem für das Wachstum von Mikroorganismen geeigneten Nähr-Gelmedium, Bewegen des Behälters, um das Gel zu verflüssigen und in der Probe enthaltene Mikroorganismen im Wesentlichen in dem Gel-Medium zu verteilen, Setzenlassen des Gel-Mediums in dem Behälter und Überwachen des Wachstums und/oder der Aktivität beliebiger Mikroorganismen in dem Gel-Medium, wobei das Gel-Medium thixotrop oder pseudoplastisch ist, so dass Bewegung seine Verflüssigung bewirkt.

2. Verfahren nach Anspruch 1, wobei das Gel-Medium ein halbsynthetisches oder synthetisches Polymer oder ein Mineral als Gelbildner enthält.

3. Verfahren nach Anspruch 2, wobei der Gelbildner eine oder mehrere der Komponenten Carboxymethylcellulose, Hydroxyethylcellulose, Hydroxypropylcellulose, Ethylcellulose, Hydroxymethylcellulose, Hydroxyalkylcellulose, ein Alginat, ein Polyethylenoxidpolymer oder Silicagel umfasst.

4. Verfahren nach Anspruch 1 oder 3, wobei das Gel-Medium als Gelbildner ein Gummi natürlichen Ursprungs umfasst.

5. Verfahren nach einem der vorangehenden Ansprüche, wobei das Gel-Medium wasserhaltig ist.

6. Verfahren nach Anspruch 4, wobei das Gummi eine oder mehrere der Komponenten Guar, Gummiarabicum, Carrageen, Stärke, Dextrin, Algin, Tragant, Pektin, Ghatti, Johannisbrot, Tara, Aloe.Chia, Leinsamen, Quittensamen, Psylliumsamen, Tamarinde, Getreidefaser (corn fibre), Karaya, Chitin, Gelatine und Xanthan umfasst.

7. Verfahren nach einem der Ansprüche 4 bis 6, wobei das thixotrope oder pseudoplastische Mittel in im Wesentlichen wasserfreier Form vorliegt und durch Hinzufügen von Wasser in das Gel-Medium transformiert wird.

8. Verfahren nach einem der vorhergehenden Ansprüche, wobei das Gel-Medium gegenüber sichtbarem Licht transparent oder transluzent ist.

9. Verfahren nach einem der vorhergehenden Ansprüche, wobei das Gel-Medium einen Indikator von mikrobiellem Metabolismus (etwa ein Tetrazoliumsalz) umfasst.

10. Verfahren nach Anspruch 9, wobei der Indikator in der Lage ist, mit mikrobiellen Zellen oder Kolonien in dem Medium zu reagieren, um eine Farbe hervorzurufen, die leicht von der gewöhnlichen Farbe des Gels unterscheidbar ist.

## Revendications

1. Procédé de contrôle de micro-organismes dans un échantillon, lequel procédé comporte les étapes d'introduction de l'échantillon dans un récipient hermétique avec un milieu de gel nutritif convenant à la croissance de micro-organismes, d'agitation du récipient afin de liquéfier le gel et de disperser sensiblement dans le milieu de gel des micro-organismes présents dans l'échantillon, à permettre au milieu de gel de se fixer dans le récipient, et de contrôle de la croissance et/ou de l'activité de tout micro-organisme dans le milieu de gel, dans lequel le milieu de gel est thixotrope ou pseudo-plastique, de telle sorte que l'agitation entraîne la liquéfaction de celui-ci.

2. Procédé selon la revendication 1, dans lequel ledit milieu de gel comprend un polymère semi-synthétique ou synthétique, ou un minéral, en tant qu'agent gélifiant.

3. Procédé selon la revendication 2, dans lequel ledit agent gélifiant comprend un ou plusieurs composés parmi carboxyméthylcellulose, hydroxyéthyl cellulose, hydroxypropyl cellulose, éthyl cellulose, hydroxyméthyl cellulose, hydroxyalkyl cellulose, un alginate, un polymère d'oxyde de polyéthylène ou un gel de silice.

4. Procédé selon la revendication 1 ou 3, dans lequel ledit milieu de gel comprend une gomme dérivée d'une source naturelle en tant qu'agent gélifiant.

5. Procédé selon l'une quelconque des revendications précédentes, dans lequel ledit milieu de gel est aqueux.

6. Procédé selon la revendication 4, dans lequel ladite gomme comprend un ou plusieurs composés parmi le guar, la gomme arabique, le carraghénane, l'amidon, la dextrine, l'algine, l'adragante, la pectine, le ghatti, la fève de caroube, la caroube, la tara, l'aloé, chia, la graine de lin, la graine de coing, la graine de psyllium, le tamarin, la fibre de maïs, le karaya, la chitine, la gélatine et le xanthane.

7. Procédé selon l'une quelconque des revendications 4 à 6, dans lequel ledit agent thixotrope ou pseudo-plastique est fourni sous forme quasiment anhydre et est transformé en milieu de gel en ajoutant de l'eau.

8. Procédé selon l'une quelconque des revendications précédentes, dans lequel ledit milieu de gel est transparent ou translucide à la lumière visible.

9. Procédé selon l'une quelconque des revendications précédentes, dans lequel ledit milieu de gel comprend un indicateur de métabolisme microbien (tel qu'un sel de tétrazolium).

10. Procédé selon la revendication 9, dans lequel ledit indicateur est capable de réagir avec des cellules ou des colonies microbiennes dans le milieu afin de générer une couleur qui se distingue aisément de la couleur habituelle du gel.
